# EUROPÄISCHE PATENTSCHRIFT

(11) **EP 0 560 092 B1**
(45) Veröffentlichungstag und Bekanntmachung des Hinweises auf die Patenterteilung: **03.12.1997**
(21) Anmeldenummer: 93102525.8
(22) Anmeldetag: 18.02.1993
(51) Int. Cl.: A61K 31/385, A61K 31/185, A61K 31/44, A61K 9/16

(54) **Tabletten, Granulate und Pellets mit hohem Gehalt an Wirkstoffen für hochkonzentrierte, feste Darreichungsformen**
Tablets, granulates and pellets with a high drug content for highly concentrated solid administration forms
Tablettes, granules et pilules à teneur élevée en principes actifs pour formes d'administration solides à haute concentration

(30) Priorität: 11.03.1992 DE 4207717
(43) Veröffentlichungstag der Anmeldung: 15.09.1993
(73) Patentinhaber: ASTA Medica Aktiengesellschaft, D-01277 Dresden (DE)
(72) Erfinder: Goede, Joachim, Dr., W-6450 Hanau 9 (DE); Engel, Jürgen, Prof., W-8755 Alzenau (DE); Hettche, Helmut, Dr., W-6057 Dietzenbach-Steinberg (DE)
(74) Vertreter: Decker, Karl-Ludwig

(56) Entgegenhaltungen:
- EP-A- 0 189 788
- EP-A- 0 427 247
- EP-A- 0 468 245

## Beschreibung

Die vorliegende Erfindung betrifft eine Arzneimittelformulierung in Tablettenform oder granulierter oder Pelletform, enthaltend beispielsweise Thioctsäure oder Flupirtin oder Mesna in granulierter oder pelletierter Form und gegebenenfalls pharmazeutisch unbedenkliche Hilfsstoffe.

Thioctsäure (α-Liponsäure) ist chemisch eine 1,2-Dithiacyclopentan-3-valeriansäure. Die Erfindung bezieht sich nicht nur auf die razemische Form, sondern ebenso auf die reine (R)- beziehungsweise (S)-Thioctsäure sowie auf Gemische aus (R)- und (S)-Thioctsäure mit beliebiger Zusammensetzung. Thioctsäure ist Bestandteil des Zellstoffwechsels und wird daher in vielen Pflanzen und tierischen Organismen gefunden. Sie wirkt als eines der Coenzyme bei der oxydativen Decarboxylierung von Pyruvat und anderen α-Ketosäuren. Thioctsäure wird seit längerer Zeit bei verschiedenen Erkrankungen eingesetzt, so unter anderem bei Lebererkrankungen, bei Leberschädigung durch Pilzvergiftung sowie bei diabetischer und alkoholischer Polyneuropathie, einer mit Stoffwechselerkrankungen einhergehenden Veränderung peripherer Nerven.

Die derzeit handelsüblichen thioctsäurehaltigen Tablettenzubereitungen enthalten maximal 200 mg Thioctsäure in einer Tablette zu 515 mg.
Zur Erleichterung der Einnahme und zur Erhöhung der Akzeptanz beim Patienten besteht der Bedarf für höher konzentrierte Thioctsäure-Tabletten mit kleinerem Format.

Mesna ist chemisch das Natriumsalz der Mercaptoethansulfonsäure. Mesna wird als Mukolytikum und zur Verhinderung der Blasentoxizität und Nephrotoxizität bei der Therapie mit Zytostatika vom Oxazaphosphorin-Typ eingesetzt.
Neben dem Natriumsalz kann auch das in EP 198 542 beschriebene Argininsalz eingesetzt werden. Flupirtin wird als Maleat zur Bekämpfung von Schmerzzuständen eingesetzt. Neben dem Maleat können auch das Hydrochlorid, das Sulfat, das Mandelat sowie weitere, pharmazeutisch verträgliche Salze verwendet werden.

Die proportionale Vervielfachung der Bestandteile in der 200 mg Thioctsäure enthaltenden Tablette führt bei Wirkstoffdosierungen von mehr als 500 mg pro Tablette bereits zu Tabletten mit Einzelgewichten von mehr als 1,2 g.
Tabletten mit solch hohem Einzelgewicht sind aufgrund ihrer Größe nur schwer schluckbar und führen zu einer Verschlechterung der Akzeptanz. Es ist erforderlich, den Hilfsstoffanteil in den höher dosierten festen Arzneiformen zu reduzieren.
Nun lassen sich jedoch thioctsäurehaltige, mesnahaltige oder flupirtinhaltige Tabletten mit reduziertem Hilfsstoffanteil mit üblichen Herstellungsverfahren nicht in befriedigender Qualität herstellen.
Höhere Konzentrationen von Thioctsäure führen zu Problemen bei der Tablettenpressung. Die Preßmassen neigen zur Anhaftung an den Preßwerkzeugen.
Darüberhinaus treten bei den Tabletten Risse parallel zur Oberfläche auf, bei bikonvexen Tabletten kommt es zum Absprengen der Kugelkappen (Deckeln).
Diese Störungen sind bedingt durch die Eigenschaften der Thioctsäure; als besonders kritisch erweist sich der niedrige Schmelzpunkt der Substanz von 60,5 °C (R, S-Thioctsäure) beziehungsweie 47 °C (R-Thioctsäure) und 46°C (S-Thioctsäure).

Die gleichen Probleme treten auf, wenn man versucht, hochkonzentrierte Mesna- oder Flupirtinmaleat-Tabletten herzustellen. Auch bei diesen Wirkstoffen treten Tablettierstörungen wie Anhaftung und Rißbildung auf, sobald der Wirkstoffanteil in der Preßmasse mehr als 45 Gewichts% beträgt.

Bei der Pelletierung stehen die Anhaftung und das Verschmieren an den Pelletiergeräten im Vordergrund. Daneben zeigen mit üblichen Hilfsstoffen in üblicher Weise angefeuchtete Pelletmassen nach der Trocknung der fertigen Pellets nur ungenügende Bindung der Pellets.

EP-A-0 420 042 beschreibt die Herstellung fester Arzneiformen mit einem hohen Gehalt an Verapamil-Hydrochlorid durch Granulation mit wenig Wasser, der Gehalt an Wasser bezogen auf Verapamil-Hydrochlorid liegt zwischen 2 und 10 Gewichts%. Auch in dem obligatorischen zweiten Granulationsschnitt des Verfahrens beträgt der Gehalt an Wasser zwischen 2 und 10 Gewichts%.

EP-A-0 468 245 beschreibt Arzneimittelformulierungen in Form von Tabletten und Granulat, welche den Wirkstoff Mesna in einer Gewichtsmenge zwischen 10 und 80 Gewichts% enthalten. Aus der Beschreibung geht hervor, daß eine wäßrige Granulation des Wirkstoffes zur Vorbereitung der Tablettierung weder durch konventionelles Anteigen noch durch Wirbelschichtgranulation gelang, da sich Mesna bei Kontakt mit Wasser sofort verflüssigt. Vorgeschlagen wird deshalb, den Wirkstoff Mesna mit einem C₁-C₄-Alkohol oder Aceton oder einer Mischung der genannten organischen Mittel mit Wasser zu granulieren. Ein solches Granulieren des Wirkstoffes mit einem organischen Mittel oder dessen Mischung mit Wasser ermöglicht erst die gute Tablettierung.

EP-A-0 427 247 offenbart Arzneimittelformulierungen in Form von Tabletten, Granulaten oder Pellets, die als Wirksubstanz R(S)-alpha-Liponsäure (Thioctsäure) enthalten. Die Tabletten können einen Wirkstoffgehalt bis zu 45 % aufweisen.

Der vorliegenden Erfindung liegt daher die Aufgabe zugrunde, Formulierungen mit hohem Anteil an Wirkstoffen zur Verfügung zu stellen, die sich einwandfrei zu Tabletten verpressen lassen. Hierzu sind die preßtechnisch ungünstigen Eigenschaffen beispielsweise der Thioctsäure, des Mesna oder des Flupirtins, die sich mit zunehmender Wirkstoffkonzentration immer stärker auswirken, zu neutralisieren.

Durch Reduktion des Anteils der Hilfsstoffe in der Tablette reduziert man gleichzeitig auch die Wahrscheinlichkeit von Unverträglichkeitsreaktionen, wie zum Beispiel auf Lactose (Deutsche Apothekerzeitung 131, 1569 (1991)).

Diese Aufgabe wird gelöst durch eine besonders intensive Granulation der Wirkstoffe mit hohen Mengen an Bindemittellösungen oder durch Granulation einer Wirkstoff/Bindemittel-Mischung mit großen Mengen an Wasser.

Neben diesen beiden Bestandteilen kann das Granulat noch gebräuchliche Tablettenhilfsstoffe wie Füll-, Spreng- und Netzmittel enthalten. Die Tablette kann neben dem Granulat noch weitere Füll-, Binde-, Spreng-, Netz-, Fließhilfsmittel, Schmiermittel und Gegenklebemittel enthalten.

Überraschend wurde gefunden, daß durch eine intensive Durchfeuchtung der zu granulierenden Masse die technologisch ungünstigen Eigenschaften der Thioctsäure und der Wirkstoffe Mesna und Flupirtin neutralisiert werden können.

Die erfindungsgemäßen Arzneimittelformulierungen enthalten zwischen 45 Gewichts% und 100 Gewichts%, vorzugsweise zwischen 75 Gewichts% und 100 Gewichts% und besonders bevorzugt zwischen 85 Gewichts% und 100 Gewichts% des Wirkstoffes Thioctsäure oder Flupirtin oder zwischen mehr als 80 Gewichts% und 100 Gewichts% Mesna. Bezogen auf die vorgelegte Feststoffmenge an Wirkstoff werden mindestens 30 Gewichts%, vorzugsweise 40 - 100 Gewichts%, insbesondere 50 - 70 Gewichts% Wasser oder wäßrige Bindemittellösungen verwendet. Bisher übliche Granulationsverfahren arbeiten mit maximal 30 Gewichts% Wasser (Der Pharma-Werker, Editio Cantor, 1970, Seite 72 - 74; Hager Handbuch der pharm. Praxis, 4. Auflage 1971, Band 7 a, Seite 712) als Granulierflüssigkeit oder Lösungsmittel für die Bindemittel. Für Thioctsäure wurden bisher maximal 15 Gewichts% Wasser als Granulationsflüssigkeit verwendet.

In List, Arneiformenlehre, Stuttgart (1985), Seite 86 wird der Feuchtigkeitsgehalt der zu granulierenden Masse folgendermaßen beschrieben:

"Im allgemeinen gilt, daß eine Masse dann den richtigen Feuchtigkeitsgehalt hat, wenn sie sich beim Zusammendrücken mit der Hand zu einem Ball formen läßt und beim Verlassen der Hand nicht sofort wieder zerfällt, andererseits sich aber gut zwischen den Fingern verreiben läßt, ohne zu schmieren". In Voigt, Lehrbuch der pharmazeutischen Technologie, Weinheim (1987), Seite 158 wird der Grad der Feuchtigkeit des Granulats mit "erdfeucht" beschrieben.
Als Befeuchtungsmittel können Alkohole mit 1 - 4 Kohlenstoffatomen, Ester aus niederen organischen Säuren und niederen organischen Alkoholen mit insgesamt bis zu 6 Kohlenstoffatomen verwendet werden, zum Beispiel, Methanol, Ethanol, Isopropanol, Essigsäureethylester und besonders bevorzugt, Wasser.

Als Bindemittel können alle pharmazeutisch gebräuchlichen Bindemittel wie Cellulosederivate (zum Beispiel Ethylcellulose, Hydroxyethylcellulose, Carboxymethylcellulose, Methylcellulose, Hydroxypropvlmethylcellulose), Gelatine, Stärke, Polyglycole (mittlere Molmasse 1000 - 35000 Dalton), Polyvinylalkohole, Polyvinylpyrrolidon, Polyacrylsäure, Vinvlpyrrolidon-Vinylacetat-Copolymerisat, Alginate Saccharose oder Glucose, Polysaccharide, wie zum Beispiel natürliche Gummisorten, wie zum Beispiel Gummi Arabicum, Tragant, Pektin, Guar-Gummi in Mengen von 1 - 30 Gewichts%, vorzugsweise 5 - 20 Gewichts%, insbesondere 10 - 15 Gewichts% ,bezogen auf Wirkstoffe, eingesetzt werden (Konzentration der wässrigen Bindemittellösungen 2 - 30 Gewichts% vorzugsweise 5 - 15 % Gewichts%).

Dabei können auch gleichzeitig verschiedene Bindemittel, zum Beispiel unterschiedliche Cellulosederivate nebeneinander, eingesetzt werden. Die Bindemittel können in die trockene Pulvermischung eingearbeitet werden oder in der Granulierflüssigkeit gelöst oder dispergiert eingebracht werden.
Auch die Kombination von trocken vorgelegtem und gelöstem beziehungsweise dispergiertem Bindemittel ist geeignet.

Die feuchte Masse wird in üblicher Weise intensiv mechanisch durchgearbeitet, um eine gleichmäßige Durchfeuchtung und Verdichtung der Masse zu erzielen. Dies geschieht beispielsweise in einem Hochleistungsgranulator. Diese Geräte besitzen einen um eine vertikale Achse laufenden Mischerarm und einen mit höherer Geschwindigkeit senkrecht oder parallel zur Mischerarmachse rotierenden Zerhacker. (Gerätebeispiele sind: Diosna-Pharmamischer, Typenreihe P, Lödige-Pflugscharmischer FM mit Messerkopf-Ausrüstung, Colette- oder Fielder-Mischer-Granulator). Die Granulation erfolgt mit maximalem Energieeintrag, das heißt der jeweils höchsten Umdrehungsgeschwindigkeit von Mischerflügel und Zerhacker und dauert je nach Granulator 5 - 20 Minuten. Die Granulatkonsistenz ist mit pastenartig` aber ohne sichtbar abgetrennte flüssige Phase, zu beschreiben. Im Gegensatz dazu werden die herkömmlichen Granulate im optimal feuchten Zustand als von schneeballartiger Konsistenz bezeichnet. Falls erforderlich, wird die feuchte Masse durch ein Passiergerät mit einer Ringmatrize (zum Beispiel Alexanderwerk-Reibschnitzler, Stephan-Granuliermaschine, Nica-Extruder) oder ein Granuliersieb gegeben und dabei weiter verdichtet. Durch eine besonders hohe Verdichtung lassen sich pelletartige Produkte erzielen. Unter Pellets sind sphärische oder zylinderförmige Partikel mit einem Durchmesser von 0,1 bis etwa 5 mm zu verstehen. Solche Pellets können vor der Trocknung noch auf einer sich drehenden, geriffelten Scheibe gerundet und geglättet werden (zum Beispiel im Nica-Spheronizer).

Anschließend werden das Granulat oder die Pellets in der Wirbelschicht oder auf Horden in üblicher Weise getrocknet bis auf eine Endfeuchte von unter 10, vorzugsweise unter 6 Gewichts% und insbesondere unter 3 Gewichts% (bezogen auf das Feststoffgewicht). Gegebenenfalls wird die Granulierung mit mehr als 15 Gewichts% vorzugsweise 30 - 70 Gewichts%, insbesondere 40 - 50 Gewichts% Wasser oder wässriger Bindemittellösungen wiederholt, um die Verdichtung des Granulates und die Bindung innerhalb der Granulatkörner zu steigern.
Der Gehalt an Bindemittel beträgt 2 - 30 Gewichts%, vorzugsweise 5 - 15 Gewichts%, bezogen auf die eingesetzte Lösungsmittelmenge.

Falls erforderlich, werden das Granulat oder die Pellets mit Füll-, Binde-, Spreng-, Netz-, Fließhilfsmittel, Schmiermittel und/oder Gegenklebemittel versetzt. Als Füllmittel können beispielsweise eingesetzt werden:
Cellulose, Cellulosederivate, Saccharose, Lactose, Glucose, Fructose, Calciumphosphate, Calciumsulfate, Calciumcarbonate, Stärke, modifizierte Stärke, Zuckeralkohole wie Sorbitol oder Mannitol.

Als Bindemittel eignen sich beispielsweise Cellulosederivate (zum Beispiel Ethylcellulose, Methylcellulose, Hydroxyethylcellulose, Carboxymethylcellulose, Hydroxypropylmethylcellulose), Gelatine, Stärke, modifizierte Stärke, Polyglycole (mittlere Molmasse 1000 - 35000 Dalton), Polyvinylalkohole` Polyvinylpyrrolidon, Polyacrylsäure, Vinylpyrrolidon-Vinylacetat-Copolymerisate, Alginate Saccharose, Glucose, Polysaccharide.
Als Sprengmittel können beispielsweise eingesetzt werden: Stärke, modifizierte Stärke, Cellulose, Cellulosederivate, Alginate oder quervernetztes Polyvinylpyrrolidon.

Als Netzmittel sind beispielsweise einsetzbar:
Natriumdioctylsulfosuccinat, Natriumlaurylsulfat, Polysorbate oder Polyoxyethylen-Stearinsäureester. Als Fließhilfsmittel eignen sich beispielsweise:
Kolloidales Siliciumdioxid, Talkum oder Magnesiumstearat.
Als Schmiermittel können beispielsweise eingesetzt werden: Magnesiumstearat, Calciumstearat, D,L-Leucin, Talkum, Stearinsäure, Polyglycole (mittlere Molmasse 3000 - 35000), Fettalkohole oder Wachse.
Als Gegenklebemittel sind beispielsweise einsetzbar: Stärke, Talkum, Magnesiumstearat, Calciumstearat oder D,L-Leucin.

Die so hergestellte Mischung wird in üblicher Weise zu Tabletten verpreßt. Dabei kann es von Vorteil sein, die Preßmassentemperatur vor der Verpressung unter die Raumtemperatur abzusenken. Die Preßmassentemperatur kann 0 °C - 30 °C, vorzugsweise 5 °C - 20 °C, insbesondere 8 °C - 15 °C betragen.

Die Pellets werden entweder zu Tabletten gepreßt oder in Hartgelatinekapseln oder Beutel gefüllt.

Kennzeichnend und entscheidend für das Verfahren sind die intensive mechanische Bearbeitung und die große Menge Granulierflüssigkeit. Durch eine Vorkühlung der Preßmasse (24 Stunden bei + 8 °C) werden die Preßeigenschaften der Tablettenmasse noch verbessert.

### Beispiele:

### Beispiel 1

### Granulat mit 300 mg Thioctsäure auf 318 mg Granulat

Maisstärke wird mit Wasser in üblicher Weise (List Arzneiformenlehre, Stuttgart (1985), Seite 88) zu Maisstärkekleister verarbeitet.
1500 g Thioctsäure werden mit 880 g eines 10 %igen wässrigen Maisstärkekleister in einem Diosna P10-Mischergranulator 10 Minuten befeuchtet (maximaler Energieeintrag: Mischer 433 U/min, Zerhacker 3000 U/min). Die feuchte Masse wird durch ein Sieb mit 2 mm Maschenweite gestrichen und auf Trockenhorden ausgebreitet in einem Umlufttrockenschrank mit einer Zulufttemperatur von 40 °C bis auf eine relative Feuchte von 25 - 30 % getrocknet. Das trockene Granulat wird durch ein Sieb der Maschenweite 0,8 mm gegeben. Das Granulat kann gegebenenfalls mit einem magensaftlöslichen, magensaftpermeablen oder magensaftunlöslichen Überzug versehen und in Kapseln oder Beutel abgefüllt werden.

### Beispiel 2

### Tabletten mit 300 mg Thioctsäure auf 319 mg Tablettengewicht.

1588 g des Granulates aus Beispiel 1 werden mit 6 g Magnesiumstearat versetzt und zu bikonvexen Tabletten mit einem Gewicht von 319 mg, einem Durchmesser von 10 mm und einem Wölbungsradius von 8 mm gepreßt. Die Tabletten sind glatt, glänzend und ohne Risse. Anhaftungen der Tablettenmasse an den Preßwerkzeugen sind nicht erkennbar.

Die Tabletten zerfallen innerhalb von 2 Minuten im Zerfallstester des DAB 9 (Prüfflüssigkeit: Wasser, 37 °C).

Gegebenenfalls können die Tabletten nach üblichen Methoden mit einem magensaftlöslichen oder magensaftresistenten Filmüberzug versehen werden.

### Beispiel 3

### Granulat mit 300 mg Thioctsäure auf 330 mg Granulat

1500 g Thioctsäure werden mit 150 g Hydroxypropylcellulose gemischt. Die Mischung wird mit 900 g gereinigtem Wasser in einem Diosna P10-Mischergranulator 12 Minuten durchfeuchtet (maximaler Energieeintrag: Mischer 433 U/min, Zerhacker Stufe 3000 U/min).

Die feuchte Masse wird durch eine Granuliermaschine (Stephan KG-150P) mit einem Ringzylinder, Lochweite 2 mm, gegeben. Das feuchte Granulat wird in einem Wirbelschicht-Trockner (Glatt WSG 3/5) bei einer Zulufttemperatur von 30 - 40 °C bis auf eine relative Feuchte von 25 - 30 % getrocknet. Das trockene Granulat wird durch ein Sieb der Maschenweite 0,8 mm gegeben.

Das Granulat kann in Kapseln oder Beutel abgefüllt werden.

### Beispiel 4

### Tabletten mit 300 mg Thioctsäure auf 331 mg Tablettengewicht

1650 g des Granulates aus Beispiel 3 werden mit 6 g Magnesiumstearat versetzt und zu Oblong-Tabletten mit einem Gewicht von 331 mg und den Abmessungen 13 x 6 mm, Wölbungsradius 4,5 mm gepreßt. Die Tabletten sind glatt, glänzend und ohne Risse und enthalten 300 mg Thioctsäure. Anhaftungen der Tablettenmasse an den Preßwerkzeugen sind nicht erkennbar.
Die Tabletten zerfallen innerhalb von 2 Minuten im Zerfallstester des DAB 9 (Prüfflüssigkeit: Wasser, 37 °C).
Gegebenenfalls können die Tabletten nach üblichen Methoden mit einem magensaftpermeablen, magensaftlöslichen oder magensaftresistenten Filmüberzug versehen werden.

### Beispiel 5

### Granulat mit 800 mg Thioctsäure auf 825 mg Granulat

1800 g Thioctsäure werden mit 720 g einer 5 %igen Gelatinelösung in einem Diosna P10-Mischergranulator 15 Minuten gemischt (Mischer 433 U/min, Zerhacker 3000 U/min). Die feuchte Masse wird durch ein Sieb der Maschenweite 3,15 mm gestrichen und in einem Wirbelschicht-Trockner (Glatt WSG 3/5) bei einer Zulufttemperatur von 30 - 35 °C bis auf eine relative Feuchte von 30 - 35 % getrocknet. Das trockene Granulat wird durch ein Sieb der Maschenweite 1,0 mm gegeben. Anschließend wird das trockene Granulat mit 432 g einer 5 %igen Gelatinelösung in einem Diosna P10-Mischergranulator 10 Minuten gemischt (Mischer 433 U/min, Zerhacker 3000 U/min). Die feuchte Masse wird in einem Wirbelschicht-Trockner (Glatt WSG 3/5) bei einer Zulufttemperatur von 25 - 35 °C bis auf eine relative Feuchte von 30 - 35 % getrocknet. Das trockene Granulat wird durch ein Sieb der Maschenweite 1,25 mm gegeben. Das Granulat kann in Kapseln oder Beutel abgefüllt werden.

### Beispiel 6

### Tabletten mit 800 mg Thioctsäure auf 835 mg Tablettengewicht

1858 g des Granulates aus Beispiel 5 werden mit 20 g Magnesiumstearat versetzt und zu Oblong-Tabletten, Größe 18 x 8 mm, Gewicht 835 mg verpreßt. Die Tabletten sind glatt, glänzend und ohne Risse und enthalten 800 mg Thioctsäure. Anhaftungen der Tablettenmasse an den Preßwerkzeugen sind auch gegen Ende der Verpressung nicht erkennbar. Die Tabletten zerfallen innerhalb von 3 - 5 Minuten im Zerfallstester des DAB 9 (Prüfflüssigkeit: Wasser, 37 °C).

### Vergleichsbeispiel 7

### Tabletten mit 300 mg Thioctsäure auf 331 mg Tablettengewicht

1500 g Thioctsäure werden mit 150 g Hydroxypropylcellulose gemischt. Die Mischung wird mit 250 g gereinigtem Wasser in einem Diosna P10-Mischergranulator 3 Minuten gemischt (Mischer 215 U/min, ohne Zerhacker, anschließend 2 Minuten Mischer 215 U/min, Zerhacker 1500 U/min).

Die erdfeuchte Masse wird durch eine Granuliermaschine (Stephan KG-150P) mit einem Ringzylinder, Lochweite 2 mm, gegeben. Das feuchte Granulat wird in einem Wirbelschicht-Trockner (Glatt WSG 3/5) bei einer Zulufttemperatur von 30 - 40 °C bis auf eine relative Feuchte von 25 - 30 % getrocknet. Das trockene Granulat wird durch ein Sieb der Maschenweite 0,8 mm gegeben und mit 6 g Magnesiumstearat homogen gemischt und auf einer Exzenter- oder Rundläuferpresse zu Oblong-Tabletten mit einem Gewicht von 331 mg und den Abmessungen 13 x 6 mm, Wölbungsradius 4,5 mm gepreßt. Bereits nach Pressung weniger Tabletten haften die Tabletten an den Preßwerkzeugen an, lösen sich beim Ausstoß aus der Maschine nicht vollständig von den Preßwerkzeugen ab und deckeln oder zeigen Längsrisse am Steg.

### Beispiel 8

### Beispiel für hochdosierte Flupirtin Tablette

1500 g Flupirtinmaleat werden mit 150 g Hydroxypropylcellulose (L-HPC LH22) gemischt. Zu dieser Pulvermischung werden 1500 g gereinigtes Wasser gegeben und in einem Intensivmischer, Diosna P 25 mit maximalen Energieeintrag 15 Minuten gemischt (Mischer 350 U/min, Zerhacker 3000 U/min). Die feuchte Masse wird 30 Minuten bei 60°C auf Horden im Umlufttrockenschrank vorgetrocknet und durch ein Sieb der Maschenweite 3,15 mm gegeben. Das Granulat wird in einem Umlufttrockenschrank bei 50°C auf eine relative Feuchte von 30 - 35 % getrocknet und durch ein Sieb der Maschenweite 1,0 mm gegeben. Nach Zugabe von 16,5 g Magnesiumstearat zu dem gesiebten Granulat wird in einem Turbula-Mischer, T10B 2 Minuten bei 30 U/min gemischt. Die Mischung wird zu biplanen Tabletten mit einem Durchmesser von 9 mm und einem Gewicht von 143 mg verpreßt. Die Masse läßt sich problemlos und ohne Anhaftung an den Preßwerkzeugen pressen.
Die Tabletten sind glatt, schwach glänzend und ohne Risse. Sie zerfallen in weniger als einer Minute im Zerfallstestgerät des DAB9 (Prüfflüssigkeit: Wasser, 37°C).

### Beispiel 9 für hochdosierte Mesna Tablette

### Tablette mit 300 mg Mesna auf 322 mg Tablettengewicht

416 g Maisstärke werden mit 2080 g Wasser in üblicher Weise zu Maisstärkekleister verarbeitet.
6000 g Mesna werden mit diesem Maisstärkekleister in einem Intensivmischer, Diosna P 25 mit maximalem Energieeintrag 12 Minuten gemischt (Mischer 350 U/min, Zerhacker 3000 U/min). Die feuchte Masse wird 10 Minuten bei 50°C auf Horden im Umlufttrockenschrank vorgetrocknet und durch ein Sieb der Maschenweite 3,15 mm gegeben. Nach weiterer achtstündiger Trocknung bei 50°C im Umlufttrockenschrank wird das Granulat durch ein Sieb der Maschenweite 0,8 mm gegeben. Nach Zugabe von 13 g Magnesiumstearat zu dem gesiebten Granulat wird in einem Turbula-Mischer, T10B 5 Minuten bei 30 U/min gemischt. Die Mischung wird zu Tabletten mit einem Durchmesser von 9 mm, Wölbungsradius 12,5 mm und einem Gewicht von 322 mg verpreßt. An den Preßwerkzeugen ist auch nach längerer Maschinenlaufzeit kein Anhaften oder Belagbildung durch die Preßmasse erkennbar. Die Tabletten sind glatt, schwach glänzend und ohne Risse. Sie zerfallen innerhalb von 5 - 7 Minuten im Zerfallstestgerät des DAB9 (Prüfflüssigkeit: Wasser, 37°C).

## Patentansprüche

1. Arzneimittelformulierung in Form von Tabletten, Granulaten oder Pellets enthaltend Thioctsäure (α-Liponsäure) oder Flupirtinmaleat, dadurch gekennzeichnet, daß der Wirkstoffgehalt mehr als 45 Gewichts% beträgt.

2. Arzneimittelformulierung in Form von Tabletten, Granulaten oder Pellets enthaltend Thioctsäure (α-Liponsäure) oder Flupirtinmaleat, dadurch gekennzeichnet, daß der Wirkstoffgehalt an Thioctsäure oder Flupirtinmaleat mehr als 75 Gewichts% beträgt.

3. Arzneimittelformulierung in Form von Tabletten, Granulaten oder Pellets enthaltend Thioctsäure (α-Liponsäure) oder Mesna oder Flupirtinmaleat, dadurch gekennzeichnet, daß der Wirkstoffgehalt mehr als 85 Gewichts% beträgt.

4. Arzneimittelformulierung in Form von Tabletten, Granulaten oder Pellets, enthaltend Thioctsäure (α-Liponsäure), dadurch gekennzeichnet, daß das zur Herstellung der Tabletten erforderliche Granulat durch eine intensive Durchfeuchtung mit mehr als 30 Gewichts% Wasser, bezogen auf die eingesetzte Menge an Feststoffen, hergestellt wird.

5. Arzneimittelformulierung in Form von Tabletten, Granulaten oder Pellets, enthaltend Mesna, dadurch gekennzeichnet, daß das zur Herstellung der Tabletten erforderliche Granulat durch eine intensive Durchfeuchtung mit mehr als 30 Gewichts% Wasser, bezogen auf die eingesetzte Menge an Feststoffen, hergestellt wird.

6. Arzneimittelformulierung in Form von Tabletten, Granulaten oder Pellets, enthaltend Flupirtin-Maleat (Maleat des 2-Amino-3-ethoxycarbonyl-amino-6-(4-fluor)-benzylaminopyridins), dadurch gekennzeichnet, daß das zur Herstellung der Tabletten erforderliche Granulat durch eine intensive Durchfeuchtung mit mehr als 30 Gewichts% Wasser, bezogen auf die eingesetzte Menge an Feststoffen, hergestellt wird.

7. Verfahren zur Herstellung einer Arzneimittelformulierung gemäß den Ansprüchen 1-6, dadurch gekennzeichnet, daß das Granulat durch wiederholte Durchfeuchtung mit maximalem Energieeintrag der hierfür vorgesehenen üblichen Geräte mit mehr als 15 Gewichts% Wasser und jeweils anschließender Trocknung hergestellt und gegebenenfalls weiter zu Pellets oder Tabletten mit den hierfür üblichen Methoden weiterverarbeitet wird.

8. Verfahren gemäß Anspruch 7, dadurch gekennzeichnet, daß der Wirkstoff Thioctsäure ist.

9. Verfahren gemäß Anspruch 7, dadurch gekennzeichnet, daß der Wirkstoff Mesna ist.

10. Verfahren gemäß Anspruch 7, dadurch gekennzeichnet, daß der Wirkstoff Flupirtinmaleat ist.

11. Arzneimittelformulierung in Form von Granulatkörpern oder Pellets, dadurch gekennzeichnet, daß der Wirkstoff oder das Gemisch aus Wirkstoff und einem oder mehreren Bindemitteln mit mehr als 30 Gewichts% Wasser, bezogen auf das Trockengewicht des Feststoffes, und mit maximalem Energieeintrag einmal oder mehrmals granuliert wird.

12. Arzneimittelformulierung nach Anspruch 11, dadurch gekennzeichnet, daß das Granulat oder die Pellets Thioctsäure enthalten.

13. Verfahren zur Herstellung von Granulat oder Pellets, dadurch gekennzeichnet, daß der Wirkstoff oder das Gemisch aus Wirkstoff und einem oder mehreren Bindemitteln mit mehr als 30 Gewichts% Wasser, bezogen auf die eingesetzte Menge an Feststoffen, mit den hierfür üblichen Methoden unter maximalem Energieeintrag der hierfür üblicherweise vorgesehenen Geräte granuliert oder pelletiert wird und das erhaltene Granulat oder die Pellets gegebenenfalls zu Tabletten weiterverarbeitet werden.

## Claims

1. Medicinal formulation in the form of tablets, granules or pellets, containing thioctic acid (α-lipoic acid) or flupirtine maleate, characterised in that the content of active ingredients is more than 45 wt.%.

2. Medicinal formulation in the form of tablets, granules or pellets, containing thioctic acid (α-lipoic acid) or flupirtine maleate, characterised in that the content of the active ingredients thioctic acid or flupirtine maleate is more than 75 wt.%.

3. Medicinal formulation in the form of tablets, granules or pellets, containing thioctic acid (α-lipoic acid) or mesna or flupirtine maleate, characterised in that the content of active ingredients is more than 85 wt.%.

4. Medicinal formulation in the form of tablets, granules or pellets, containing thioctic acid (α-lipoic acid), characterised in that the granular material required for the production of the tablets is prepared by an intensive thorough moistening with more than 30 wt.% water, based on the quantity of solids used.

5. Medicinal formulation in the form of tablets, granules or pellets, containing mesna, characterised in that the granular material required for the production of the tablets is prepared by an intensive thorough moistening with more than 30 wt.% water, based on the quantity of solids used.

6. Medicinal formulation in the form of tablets, granules or pellets, containing flupirtine maleate (maleate of 2-amino-3-ethoxycarbonylamino-6-(4-fluoro)benzylaminopyridine), characterised in that the granular material required for the production of the tablets is prepared by an intensive thorough moistening with more than 30 wt.% water, based on the quantity of solids used.

7. A process for the production of a medicinal formulation according to claims 1 to 6, characterised in that the granular material is prepared by repeated thorough moistening, at the maximum energy input of the conventional equipment provided for this, with more than 15 wt.% water and subsequent drying in each case, and is optionally further processed into pellets or tablets using the conventional methods for this.

8. A process according to claim 7, characterised in that the active ingredient is thioctic acid.

9. A process according to claim 7, characterised in that the active ingredient is mesna.

10. A process according to claim 7, characterised in that the active ingredient is flupirtine maleate.

11. Medicinal formulation in the form of granules or pellets, characterised in that the active ingredient or the mixture comprising active ingredient and one or more binders is granulated once or several times with more than 30 wt.% water, based on the dry weight of the solid, and with maximum energy input.

12. Medicinal formulation according to claim 11, characterised in that the granular material or the pellets contain thioctic acid.

13. A process for the production of granular material or pellets, characterised in that the active ingredient or the mixture comprising active ingredient and one or more binders is granulated or pelletised with more than 30 wt.% water, based on the quantity of solids used, using the conventional methods for this and at the maximum energy input of the equipment conventionally provided for this, and the granular material or the pellets obtained are optionally further processed into tablets.

## Revendications

1. Formulation de médicament sous forme de comprimés, de granulés ou de pilules renfermant de l'acide thioctique (acide α-lipoïque) ou du maléate de flupirtine,
caractérisée en ce que
la teneur en principe actif s'élève à plus de 45 % en poids.

2. Formulation de médicament sous la forme de comprimés, de granulés ou de pilules renfermant de l'acide thioctique (acide α-lipoïque) ou du maléate de flupirtine,
caractérisée en ce que
la teneur du principe actif en acide thioctique ou en maléate de flupirtine s'élève à plus de 75 % en poids.

3. Formulation de médicament sous la forme de comprimés, de granulés ou de pilules renfermant de l'acide thioctique (acide α-lipoïque) ou du MESNA ou du maléate de flupirtine,
caractérisée en ce que
la teneur du principe actif s'élève à plus de 85 % en poids.

4. Formulation de médicament sous la forme de comprimés, de granulés ou de pilules renfermant de l'acide thioctique (acide α-lipoïque),
caractérisée en ce que
le granulé nécessaire pour la fabrication des comprimés est produit par une imprégnation intensive avec plus de 30 % en poids d'eau, rapporté à la quantité mise en oeuvre de produits solides.

5. Formulation de médicament sous la forme de comprimés, de granulés ou de pilules renfermant du MESNA,
caractérisée en ce que
le granulé nécessaire pour la fabrication des comprimés est produit par une imprégnation intensive avec plus de 30 % en poids d'eau, rapporté à la quantité mise en oeuvre de produits solides.

6. Formulation de médicament sous la forme de comprimés, de granulés ou de pilules renfermant du maléate de flupirtine (maléate de 2-amino-3-(éthoxycarbonyl-amino)6-(4 fluoro)benzylaminopyridine),
caractérisée en ce que
le granulé nécessaire pour la fabrication des comprimés est produit par une imprégnation intensive avec plus de 30 % en poids d'eau, rapporté à la quantité mise en jeu de produits solides.

7. Procédé de production d'une formulation de médicament conformément aux revendications 1 à 6,
caractérisé en ce que
le granulé est fabriqué par imprégnation répétée avec l'apport d'énergie maximal des appareils usuels prévus pour cela, avec plus de 15 % en poids d'eau, et séchage à chaque fois consécutif, et est transformé ultérieurement en pilules ou en comprimés avec les procédés usuels pour cela.

8. Procédé conformément à la revendication 7,
caractérisé en ce que
le principe actif est l'acide thioctique.

9. Procédé conformément à la revendication 7,
caractérisé en ce que
le principe actif est le MESNA.

10. Procédé conformément à la revendication 7,
caractérisé en ce que
le principe actif est le maléate de flupirtine.

11. Formulation de médicament sous la forme de solides, de granulés ou de pilules,
caractérisée en ce que
le principe actif ou le mélange à base de principe actif et d'un ou plusieurs agents liants avec plus de 30 % en poids d'eau, rapporté au poids sec de substance solide et est granulé une fois ou plusieurs fois avec l'apport d'énergie maximal.

12. Formulation de médicament selon la revendication 11,
caractérisée en ce que
le granulé ou les pilules renferment de l'acide thioctique.

13. Procédé de fabrication de granulé ou de pellets,
caractérisé en ce que
le principe actif ou le mélange à base de principe actif et d'un ou plusieurs agents liants avec plus de 30 % en poids d'eau, rapporté à la quantité mise en oeuvre de substances solides, est granulé ou mis en pellets avec les procédés usuels pour cela avec l'apport d'énergie maximum des appareils prévus pour cela, d'une manière habituelle et en ce que le granulé ou les pellets obtenus sont transformés ultérieurement éventuellement en comprimés.
